Europäisches Patentamt

(19) European Patent Office

Office européen des brevets

(11) Veröffentlichungsnummer: **0 239 744**
B1

# (12) EUROPÄISCHE PATENTSCHRIFT

(45) Veröffentlichungstag der Patentschrift:
18.07.90

(51) Int. Cl.⁵: **G01N 33/00, B01D 53/34**

(21) Anmeldenummer: **87101400.7**

(22) Anmeldetag: **03.02.87**

(54) **Verfahren zur Reinhaltung der Messleitungen an Emissions-Messeinrichtungen, Anwendung des Verfahrens und Gasentnahmesonde zu seiner Durchführung.**

(30) Priorität: **18.02.86 DE 3605158**

(43) Veröffentlichungstag der Anmeldung:
**07.10.87 Patentblatt 87/41**

(45) Bekanntmachung des Hinweises auf die Patenterteilung:
**18.07.90 Patentblatt 90/29**

(84) Benannte Vertragsstaaten:
**AT BE DE FR GB NL SE**

(56) Entgegenhaltungen:
**US-A- 4 127 386**

**PATENT ABSTRACTS OF JAPAN, Band 9,
Nr. 50 (P-339)[1773], 5. März 1985; &
JP-A-59 188 553 (HITACHI ZOSEN K.K.) 25.10.1984**

(73) Patentinhaber: **Siemens Aktiengesellschaft,
Wittelsbacherplatz 2, D-8000 München 2(DE)**

(72) Erfinder: **Sprehe, Josef Dipl.-Ing., Quittenweg 32a,
D-8510 Fürth(DE)**
Erfinder: **Osteroth, Ralf, Miltenbergerstr. 6,
D-8500 Nürnberg(DE)**
Erfinder: **Schott, Manfred, Wehneltstr. 3,
D-8520 Erlangen(DE)**

**Beschreibung**

Die Erfindung bezieht sich auf ein Verfahren zur Reinhaltung der Meßleitungen an Emissions-Meßeinrichtungen von Rauchgas-Reinigungsanlagen, gemäß Oberbegriff des Anspruchs 1.

Als praktische Beispiele und bevorzugte Anwendungsfälle im Rahmen der vorliegenden Erfindung seien hierzu die Rauchgasanalysen bei DENOX-Anlagen und bei Rauchgasentschwefelungsanlagen nach dem Walter-Verfahren angegeben, wo es einerseits zu Verunreinigungen der Meßleitungen und Meßgeräte durch Nebenreaktionsprodukte wie Ammoniumsulfate und Ammoniumnitrate unter anderem nach folgenden Gleichungen kommt:

$$2 SO_2 + O_2 \rightarrow 2 SO_3$$
$$SO_3 + H_2O + 2 NH_3 \rightarrow (NH_4)_2 SO_4 \text{ (Ammoniumsulfat)}$$
und
$$SO_3 + H_2O + NH_3 \rightarrow NH_4HSO_4 \text{ (Ammoniumhydrogensulfat)}$$

und natürlich durch Staub. Andererseits kommt es zu Querempfindlichkeiten chemielumineszenter und IR-spektrokopischer NO- und $NO_2$-Analysegeräte in Gegenwart von $NH_3$.

Durch die vorliegende Erfindung soll das gattungsgemäße Verfahren so ausgestaltet werden, daß die Querempfindlichkeiten beseitigt und die für die Nebenreaktionsprodukte verantwortlichen Komponenten gefiltert werden können. Insbesondere soll die gestellte Aufgabe für den Fall gelöst werden, daß der Meßgasstrom als gas- oder dampfförmige Verunreinigungen wenigsten $SO_2$ und $SO_3$ (= $SO_X$) als Komponente A und $NH_3$ als Komponente B enthält.

Erfindungsgemäß wird die gestellte Aufgabe bei einem gattungsgemäßen Verfahren durch die im Kennzeichen des Anspruchs 1 angegebenen Merkmale gelöst.

Eine bevorzugte Ausgestaltung des Verfahrens nach der Erfindung ist zur Reinhaltung der Meßleitungen an Emissions-Meßeinrichtungen von Rauchgas-Entstickungsanlagen und/oder Rauchgas-Entschwefelungsanlagen, dadurch gekennzeichnet, daß der Meßgasstrom als gas- oder dampfförmige Verunreinigungen wenigstens $SO_X$ als Komponente A und $NH_3$ als Komponente B enthält.

Das Verfahren nach den Ansprüchen 1 und 2 wird durch die Merkmale der Ansprüche 3 bis 11 in vorteilhafter Weise weitergebildet.

Gegenstand der Erfindung ist auch die Anwendung des Verfahrens nach den Ansprüchen 1 bis 7 auf die kondenswasserfreie Filterung von Meßgasen, so wie im Anspruch 12 angegeben.

Gegenstand der Erfindung ist ferner eine Gasentnahmesonde zur Durchführung des in den Ansprüchen 1 bis 11 beschriebenen Verfahrens gemäß Oberbegriff des Anspruchs 13, mit der die Aufgabe gelöst wird, die beiden Komponenten A und B bzw. $SO_X$ und $NH_3$ selektiv aus dem Meßgasstrom zu entfernen, eine möglichst hohe Filterkapazität bei entsprechend großen Wartungsintervallen bereitzustellen, die Entnahme des Rauchgases an einer einzigen Stelle für beide Filter zur Wahrung der Repräsentativität der Probeentnahme vorzunehmen und eine Beheizung von rauchgasführenden Teilen zur Vermeidung einer Bildung und/oder Ausscheidung der Reaktionsprodukte aus den Komponenten A und B bzw. insbesondere der Stoffe Ammoniumsulfat, Ammoniumhydrogensulfat, Schwefelsäure und Wasser, vor Eintritt in die Filter zu gewährleisten.

Die gestellte Aufgabe wird mit einere Gasentnahmesonde gemäß Oberbegriff des Anspruchs 13 durch die im Kennzeichen des Anspruchs 13 angegebenen Merkmale gelöst. Vorteilhafte Weiterbildungen der Gasentnahmesonde nach Anspruch 13 sind in den Ansprüchen 14 bis 22 angegeben.

Durch das erfindungsgemäße Verfahren und die Gasentnahmesonde zu seiner Durchführung werden vor allem die folgenden Vorteile erzielt:
- Die Gasentnahmesonde, insbesondere DENOX-Rauchgasentnahmesonde, kann direkt am Rauchgaskanal angeordnet sein, wodurch Verunreinigungen der Entnahmeleitung ausgeschlossen sind.
- Sollten dennoch bereits Nebenreaktionsprodukte im Rauchgasstrom nach DENOX-Anlagen vorliegen, wie Ammoniumsulfate, Ammoniumhydrogensulfate, Ammoniumnitrate und $SO_3$, so werden diese in der Rauchgasentnahmesonde zusammen mit $SO_2$ und $NH_3$ abgeschieden.
- Verhinderung der Bildung von Nebenreaktionsprodukten in der Meßleitung und in den nachgeschalteten Meßgeräten durch $SO_X$- bzw. $NH_3$-Filterung.
- Die $NH_3$- und $SO_X$-Filter sind in der Entnahmesonde parallel geschaltet (Repräsentativität der Messung).
- Die Temperatur der Entnahmesonde entspricht in etwa derjenigen der Rauchgastemperatur; dadurch wird u.a. weitgehend ein Abscheiden von Ammoniumsulfaten, Ammoniumhydrogensulfat, Schwefelsäure und Wasser vor dem Filtereintritt unterbunden.
- Die Gasentnahmesonde kann in einem kompletten DENOX-Gasanalysemeßstand, vorzugsweise einer Gasaufbereitungsanlage und typgeprüften Gasanalysegeräten, vorgeschaltet werden.
- Als $SO_X$-Filter (Herausfilterung der Komponente A) wird vorzugsweise körniges Soda ($Na_2CO_3$) eingesetzt und als $NH_3$-Filter (zur Herausfilterung der Komponente B) wird vorzugsweise (heiße) Phosphorsäure eingesetzt.

- Auf diese Weise entfällt eine Absorption der in hoher Konzentration im Rauchgas vorkommenden Stoffe Wasser oder $CO_2$, welche sonst die Filter stark belasten würden.

- Vor allem der letzterwähnte Vorteil macht das Verfahren nach der Erfindung auch anwendbar auf die kondenswasserfreie Filterung von Meßgasen im Einweg-Verfahren gemäß Anspruch 8.

Im folgenden werden anhand eines in der Zeichnung dargestellten Ausführungsbeispiels die Gasentnahmesonde nach der Erfindung und das durch sie verwirklichte Verfahren zur Reinhaltung der Meßleitungen an Emissions-Meßeinrichtungen von Rauchgas-Reinigungsanlagen noch näher erläutert. Darin zeigt:

Fig. 1 schematisch die Gasentnahmesonde, eingebaut in den Rauchgasentnahmestutzen eines Rauchgaskanals, und

Fig. 2 in einem Längsschnitt die Gasentnahmesonde im Ketail in einer der praktischen Ausführung entsprechenden konstruktiven Ausgestaltung.

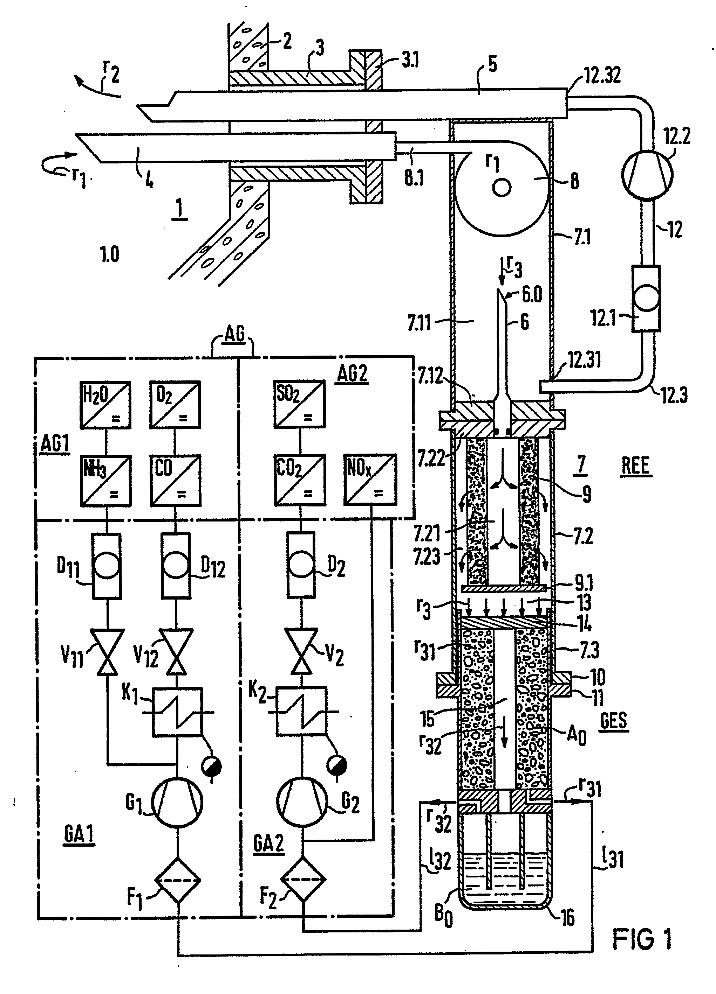

Fig. 1 zeigt schematisch, wie in den Gasweg vom Rauchgaskanal 1 bis hin zu den Gasanalysen-Meßgeräten AG die Gasentnahmesonde GES nach der Erfindung eingeschaltet ist. Der Rauchgaskanal 1, der z.B. zu einem Kohlekraft werk gehört und zu einem nicht dargestellten Abgaskamin führt, wird von einer Kanalwand 2 begrenzt, welche einen Gasentnahmestutzen 3 mit dichtend aufgesetztem Deckel 3.1 aufweist. Durch den Deckel 3.1 sind dichtend hindurchgeführt eine Rauchgas-Entnahmelanze 4 und eine Rauchgas-Rückführlanze 5, die - wie es die Pfeile symbolisieren - aus dem Rauchgasstrom eine Teilmenge $r_1$ entnehmen und eine Teilmenge $r_2$ wieder zurückführen, wobei die Teilmenge $r_2$ kleiner ist als die Teilmenge $r_1$, weil - wie noch erläutert wird - durch die kleinere Entnahmelanze 6 zu Gasanalysezwecken eine Teilmenge $r_3$ aus der Teilmenge $r_1$ entnommen wird.

Das zylindrische Gehäuse 7 der Rauchgasentnahmeeinrichtung untergliedert sich in einen Vorkammerabschnitt 7.1, welcher in seinem Inneren einen Zyklon 8 und die schon erwähnte kleinere Entnahmelanze 6 aufweist, einen Filterabschnitt 7.2 mit eingebautem hohlzylindrischen Feinstaubfilter 9, insbesondere einem Keramikfilter, und in einen Einsteckabschnitt 7.3 mit Halteflansch 10, in welchen die Gasentnahmesonde GES nach der Erfindung eingesteckt und an dem Flansch 10 mit einem eigenen Befestigungsflansch 11 dichtend befestigt ist. Der als becherförmiges Gehäuse ausgebildete Vorkammerabschnitt 7.1 enthält den schematisch angedeuteten Zyklon 8, welcher über das Einlaufrohr 8.1 mit der Rauchgasentnahmelanze 4 verbunden ist. Nach der Staubgrobabscheidung im Zyklon 8 gelangt die Rauchgasteilmenge $r_1$ in die Vorkammer 7.11, und dort wird durch die in die Vorkammer 7.11 ragende Entnahmelanze 6 eine kleine Menge $r_3$ entnommen, wogegen der größere Anteil über den Bypaß 12 wieder in die Rückführlanze 5 zurückgespeist wird. Der Bypaß 12 enthält einen Durchflußmesser 12.1 und in Reihe dazu eine Pumpe bzw. ein Gebläse 12.2 innerhalb der Bypaßleitung 12.3, wobei deren Anschlußstellen an den Vorkammerabschnitt 7.1 bei 12.31 und an das rückwärtige Ende der Rückführlanze 5 bei 12.32 dargestellt sind. Im dargestellten Beispiel wird die gesamte Rauchgasentnahmeeinrichtung REE mit ihrem rohrförmigen Gehäuse 7 von der zu diesem Zweck verstärkt ausgebildeten Rückführlanze 5 getragen; es wäre auch möglich, das Gehäuse 7 an seinem oberen Ende als Rohrknie auszuführen, welches mit dem Flansch 3.1 baulich vereinigt ist.

Die Gehäuseabschnitte 7.1 und 7.2 sind durch bodenseitige bzw. deckseitige Flanschdeckel 7.12 bzw. 7.22 dichtend miteinander verschraubt; beide Flanschdeckel werden von einem verstärkten Fußteil der Entnahmelanze 6 dichtend durchdrungen, wobei die durch diese Entnahmelanze 6 entnommene Prüfgasmenge $r_3$ in den inneren Kammerraum 7.21 und von hier über das schon erwähnte zylindrische Feinstaubfilter 9 in den Kammeraußenraum 7.23 gelangt (vgl. Strömungspfeile). Das Feinstaubfilter 9 ist bodenseitig durch einen Deckel 9.1 abgeschlossen.

In der Gasentnahmesonde GES, deren konstruktiver Aufbau weiter unten noch näher erläutert wird, wird der dem Rauchgas 1.0 an einer einzigen Entnahmestelle, und zwar 6.0 im Falle der Entnahmelanze 6 und 13 im Falle der Gasentnahmesonde GES, entnommene Meßgasstrom $r_3$ in wenigstens zwei parallele Teilströmne $r_{31}$ und $r_{32}$ unterteilt. Die beiden Teilströme $r_{31}$ und $r_{32}$ werden durch eine in Fig. 1 nicht näher dargestellte Beheizung auf eine Temperatur oberhalb des Kondensationspunktes der im Meßgas enthaltenen dampfförmigem Verunreinigungen A bzw. B beheizt. Insbesondere handelt es sich bei der Verunreinigung bzw. Komponente A um $SO_2 + SO_3$ und bei der Verunreinigung bzw. Komponente B um $NH_3$. Zum Herausfiltern der Komponente A hat sich in diesem Falle körniges Soda $Na_2CO_3$ ohne Kristallwasser als vorteilhafte Filtersubstanz $A_0$ erwiesen, und im Falle von $NH_3$ als herauszufilternde Verunreinigung bzw. Komponente B hat sich Phosphorsäure als vorteilhafte Filtersubstanz $B_0$ erwiesen. Der an der Entnahmestelle 13 noch gemeinsame Meßgasstrom $r_3$ gelangt zunächst über wenigstens ein weiteres Filter 14 in das Innere der Gasentnahmesonde GES, wobei er sich hinter diesem Filter 14 in die zwei Teilströme $r_{31}$ und $r_{32}$ aufteilt. Der erstgenannte Teilstrom $r_{31}$ durchströmt, wie gesagt, den Filter $A_0$, welcher im Falle von $SO_X$ als Komponente A zweckmäßig auf eine Temperatur von etwa 350° C aufgeheizt wird. Am Ende dieser Filterstrecke $A_0$ verläßt der erste Teilstrom $r_{31}$ diese und tritt in die Meßgasleitung $l_{31}$ über. Der zweite Teilstrom $r_{32}$ fließt zunächst durch den Zentralkanal 15 der Gasentnahmesonde GES, also im Bypaß zur Filterstrecke $A_0$, und tritt dann am unteren Ende der Gasentnahmesonde GES in einen Behälter 16 über, welcher im Falle von $NH_3$ als herauszufilternde Verunreinigung bzw. Komponente A heiße Phosphorsäure als Filtersubstanz $B_0$ enthält, welche zweckmäßig auf einer Temperatur von etwa 60° - 70° C gehalten wird. Nach Durchströmen der Filtersubstanz $B_0$ in mindestens zwei gegenläufigen Strömungspfaden verläßt der zweite Teilstrom $r_{32}$ den Behälter 16 an seinem Ende über die Meßgasleitung $l_{32}$.

Nach Durchströmen der Gasentnahmesonde GES ist mithin die Meßgasleitung $l_{31}$ $SO_X$-frei, und die andere Meßgasleitung $l_{32}$ ist $NH_3$-frei. Beide Meßgasleitungen münden in je eine Gasaufbereitungsstrecke GA1 bzw. GA2, und die in diesen Gasaufbereitungsstrecken aufbereiteten Meßgas-Teilströme gelangen dann in je eine Gasanalyseeinheit AG1 bzw. AG2. Die Gasanalyseeinheit AG1 ist der Gasaufbereitungsstrecke GA1 nachgeschaltet und enthält Analyse-Meßgeräte zur quantitativen Analyse von $H_2O$, $NH_3$, $O_2$ und CO. Die Gasanalyseeinheit AG2 ist der Gasaufbereitungsstrecke GA2 nachgeschaltet und enthält Analysemeßgeräte zur quantitativen Analyse von $SO_2$, $CO_2$ und $NO_X$. Schaltung und Arbeitsweise der Gasaufbereitungsstrecken GA1, GA2 und der Gasanalyseeinheiten AG1 und AG2 im einzelnen sind im Rahmen der vorliegenden Erfindung nicht von Belang; erwähnt sei lediglich, daß innerhalb der Gasaufbereitungsstrecke GA1 bzw. GA2 mit $F_1$ bzw. $F_2$ Filter bezeichnet sind, mit $G_1$ bzw. $G_2$ Meßgasgebläse, mit $K_1$ bzw. $K_2$ Kühler, mit $V_{11}$, $V_{12}$ bzw. $V_2$ Ventile und mit $D_{11}$, $D_{12}$ bzw. $D_2$ Durchflußmesser.

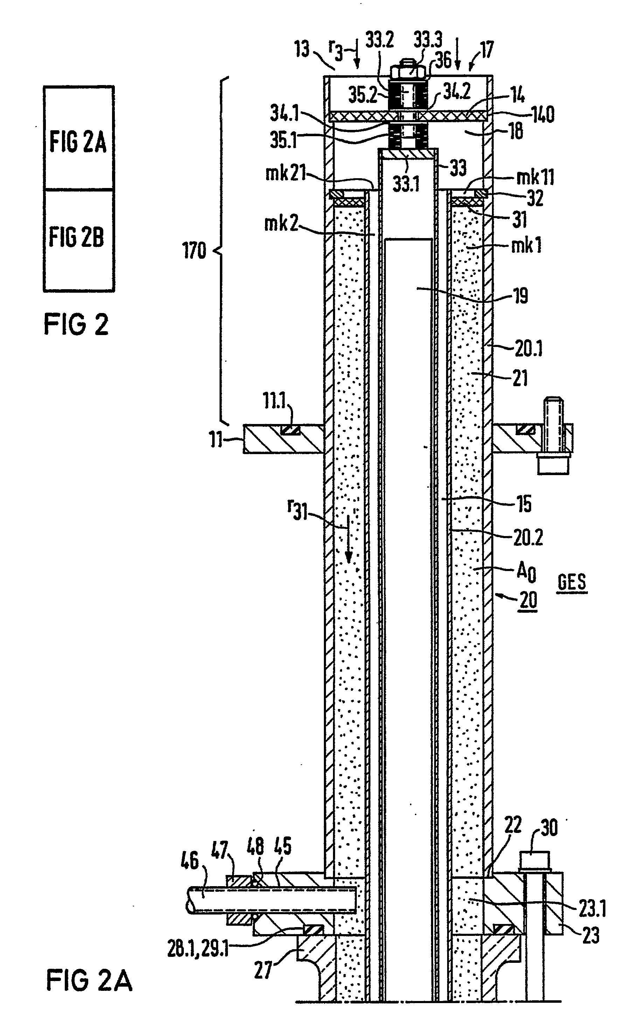

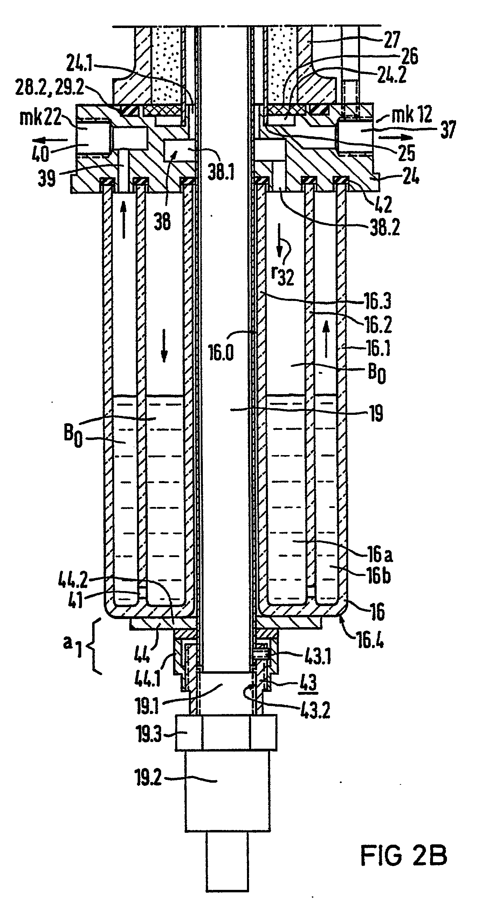

Fig. 2 zeigt nun konstruktive Details der Gasentnahmesonde GES aus Fig. 2 mit ihrem Befestigungsflansch 11, ihrem Staubfilter 14, ihrem Zentralkanal 15 und ihrem Filterbehälter 16. Die Gasentnahmesonde GES stellt inssamt einen Hohlkörper dar, mit wenigstens zwei internen, zueinander parallel geschalteten ersten und zweiten Meßgaskanälen mk1 und mk2, wobei im Meßgaskanal mk1 der Meßgas-Teilstrom $r_{31}$ und im Meßgaskanal mk2 der MeßgasTeilstrom $r_{32}$ strömt (vgl. Strömungspfeile). An beiden Enden der Meßgaskanäle befinden sich jeweils Gaseintrittsöffnungen mk11 bzw. mk21 und Gasaustrittsöffnungen mk12 bzw. mk22. Die Gasentnahmesonde GES ist mit dem Eintrittsende 17 ihres Hohlkörpers in den Gasstrom der Rauchgasleitung, sowie in Fig. 1 schematisch dargestellt, eintauchend montierbar, wobei das Eintauchende, das bis zum Flansch 11 reicht, mit 170 bezeichnet ist. Innerhalb des Eintrittsendes 17 ist eine Gaseintrittskammer 18 vorgesehen, welche auf ihrer Zuströmseite über wenigstens ein Staubfilter 14 mit dem Rauchgasleitungs-Innenraum 13 (Fig. 1) und auf ihrer Abströmseite mit den Gaseintrittsöffnungen mk11, mk21 beider Meßgaskanäle mk1 bzw. mk1 kommuniziert.

Im ersten Meßgaskanal mk1 ist das schon erwähnte Filter $A_0$ zum Herausfiltern der Komponente A (insbesondere $SO_2$) und im zweiten Meßgaskanal mk2 ist ein zweites Filter $B_0$ zum Herausfiltern der Komponente B, insbesondere $NH_3$, angeordnet. Die Strömungsräume und/oder die Wandungen beider Meßgaskanäle mk1, mk2 stehen in wärmeübertragenden Kontakt mit einer Heizeinrichtung 19, welche die durch die Meßgaskanäle mk1, mk2 strömenden Teilströme $r_{31}$, $r_{32}$ auf eine Temperatur oberhalb der höchsten Kondensationstemperatur der zu filternden Komponenten A bzw. B hält, wie anhand von Fig. 1 bereits erläutert.

Im einzelnen weist der Hohlkörper der Gasentnahmesonde GES einen doppelwandigen Hohlzylinder 20 auf, mit äusserer Ringwand 20.1 und einer radial dazu beabstandeten inneren Ringwand 20.2, wobei durch letztere das schon erwähnte Zentralkanalstück 15 begrenzt wird und durch beide Ringwände 20.1, 20.2 ein Ringkanalstück 21 begrenzt wird. Der Hohlzylinder 20 besteht aus korrosionsbeständigem Stahl; seine äußere Ringwand 20.1 ist durch eine Ringschweißnaht 22 gleichachsig zur Innenbohrung 23.1 eines ringförmigen Zwischenflansches 23 an diesen angeschweißt und endet dort. Die innere Ringwand 20.2 dagegen durchdringt die zentrale Öffnung 23.1 des Zwischenflansches 23 und erstreckt sich bis zu einem koaxial und mit Abstand zum Zwischenflansch 23 angeordneten Anschlußflansch 24, der ebenfalls ringförmig ausgeführt ist und eine zentrale Öffnung 24.1 aufweist. Die innere Ringwand 20.2 ist an der inneren Begrenzungswand eines Ringkanals 24.2 des Anschlußflansches 24 bei 25 durch eine Ringschweißnaht angeschweißt. Der Ringkanal 24.2, der in den Anschlußflansch 24 auf seiner dem Zwischenflansch 23 zugewandten Stirnseite eingebracht ist, ist durch eine in eine entsprechende Ringaussparung eingesetzte Filterscheibe 26 abgedeckt. Zwischen den einander zugewandten Stirnseiten des Zwischenflansches 23 einerseits und des Anschlußflansches 24 andererseits ist ein hohlzylindrischer Schauglaskörper 27 eingespannt der ein Profil als Körper gleicher Festigkeit aufweist mit verstärkten Enden und der mit seinen beiden Stirnflächen gegen Dichtungsringe 28.1 und 28.2 unter elastischer Deformation letzterer gepreßt wird, wobei die Dichtungsringe 28.1, 28.2, insbesondere O-Ringe, in entsprechenden Ringnuten 29.1 und 29.2 der beiden einander zugewandten Stirnflächen des Zwischenflansches 23 bzw. des Anschlußflansches 24 sitzen. Die beiden Flansche 23, 24 sind durch entsprechende achsparallele Flanschschrauben 30, die über einen Lochkreis beider Flansche über deren Umfang verteilt zu denken sind, fest und dichtend miteinander verspannt. Der Schauglaskörper 27 gestattet eine Beobachtung der zwischen ihm und der inneren Ringwand 20.2 angeordneten Filtermasse $A_0$, d.h. einen Aufschluß über deren Beladungszustand, so daß bei den Inspektionen sehr schnell und einfach von außen erkennbar ist, ob die Filtermasse $A_0$ ausgewechselt werden muß.

An seinem oberen Ende ist das Ringkanalstück 21 des Hohlzylinders 20, d.h. die in ihm enthaltene Säule aus dem Filtermaterial $A_0$, durch eine ringscheibenförmige Filterplatte 31 abgedeckt, die in ihrer Lage durch einen anliegenden Sicherungsring 32, welcher in einer Ringnut am Innenumfang der Außenwand 20.1 sitzt, abgesichert ist. Die Eintrittskammer 18, welche der Filterplatte 14 nachgeschaltet ist, mündet auf diese Weise in die erste Meßgaskanalstrecke mk1 über die Filterplatte 31, die die Gaseintrittsöff-

nung mk11 bildet; dagegen mündet die Eintrittskammer 18 in die zweite Meßgaskanalstrecke mk2 über den eingangsseitigen Ringspalt des Zentralkanalstückes 15, zugleich die Eintrittsöffnung mk21, welcher sich zwischen der inneren Ringwand 20.2 und einer zentrisch angeordneten Fingerhuthülse 33 auftut. Die Fingerhuthülse 33 dient zur leicht einsteckbaren Aufnahme eines Stabheizkörpers 19, wie weiter unten noch erläutert; ihr Boden 33.1 weist einen Schraubenbolzen 33.2 auf, welcher die mechanisch stabile Filterscheibe 14 in einer zentrischen Bohrung durchdringt, wobei zwischen dem Boden 33.1 und der Unterseite der Filterscheibe 14 ein erstes, über eine Unterlegscheibe 34.1 abgedecktes Tellerfederpaket 35.1 eingefügt ist und auf der Oberseitre der Filterscheibe 14 dementsprechend ein ebenfalls von dem Schraubenbolzen 33.2 durchdrungenes zweites Tellerfeder-Teilpaket 35.2 angeordnet ist, welches zur Filterscheibe 14 durch eine Unterlegscheibe 34.2 abgedeckt ist. An der Oberseite des zweiten Tellerfeder-Teilpaketes 35.2 ist dann unter Zwischenschaltung einer weiteren Unterleg- bzw. Beilagscheibe 36 eine Spannmutter 33.3 auf den Gewindebolzen 33.2 aufgeschraubt. Da die Fingerhuthülse 33 an ihrem unteren Ende eine axiale Spannkräfte ausübende Verschraubung aufweist, wie noch erläutert wird, so dient die federelastische Festlegung der Fingerhuthülse 33 über ihre Tellerfederpakete, die beschriebene Verschraubung 33.2, 33.3 und die Filterscheibe 14 mit ihrem Ringschultersitz 140 am Innenumfang der Außenwand 20.1 als ein Widerlager.

Aus Fig. 2 ist also ersichtlich, daß der Hohlkörper ein doppelwandiger Hohlzylinder 20 ist, mit einem Ringkanalstück 21 und einem Zentralkanalstück 15, welche beiden Kanalstücke durch die innere Ringwand 20.2 voneinander getrennt sind und dabei die zueinander parallel geschalteten und konzentrisch verlaufenden Meßgaskanalstrecken des ersten bzw. zweiten Meßgaskanals mk1 bzw. mk2 bilden. Dabei bildet der Hohlzylinder 20 mit seinem Ringkanalstück 21 eine Filterstrecke des ersten Meßgaskanals mk1. Das Ringkanalstück 21 setzt sich durch den Zwischenflansch 23 fort sowie durch den Schauglaskörper 27 und endet in dem dichtend mit dem Schauglaskörper 27 verbundenen Anschlußflansch 24. Das erwähnte Ringkanalstück geht dann über die zwischengeschaltete Filterplatte 26 des Anschlußflansches 24 in erste interne Umleitkanäle in Form des Ringkanals 24.2 über, von dem dann seitlich ein erster auslaßseitiger Anschlußkanal 37 abgeht, der einen nicht näher bezeichnete Anschlußbohrung mit Innengewinde zum gasdichten Anschluß von Nippeln externer Meßgasleitungen aufweist. Dieser erste auslaßseitige Anschlußkanal 37 weist außer seinem radial verlaufenden Kanalteil noch einen schräg verlaufenden Kanalteil auf, mit dem er den Ringkanal 24.2 anschneidet bzw. mit diesem kommuniziert.

Auch das Zentralkanalstück 15, welches einen Teil des zweiten Meßgaskanals mk2 bildet, endet in dem Anschlußflansch 24. Es ist über zweite interne Umleitkanäle 38 des Anschlußflansches 24 mit einer Filterstrecke $B_0$ des zweiten Meßgaskanals mk2 verbunden, welche auf der dem doppelwandigen Hohlzylinder 20 abgewandten Seite des Anschlußflansches 24 innerhalb eines hohlzylindrischen, dichtend mit dem Anschlußflansch 24 verbundenen Behälter 16 angeordnet ist. Dieser Behälter 16 ist in seinem geodätisch am höchsten liegenden Bereich über dritte anschlußflansch-interne Umleitkanäle 39 mit einem zweiten, seitlich abgehenden auslaßseitigen Anschlußkanal 40 des Anschlußflansches 24 verbunden, welcher zugleich die Gasaustrittsöffnung mk22 bildet, ebenso wie der Anschlußkanal 37 die Gasaustrittsöffnung mk12 bildet.

Der Behälter 16 besteht bevorzugt ebenfalls aus Schauglas entsprechender Wandstärke. Das Filtermedium $B_0$ besteht, wie erwähnt, im dargestellten Ausführungsbeispiel bevorzugt aus einem Phosphorsäurebad, welches den Behälter bevorzugt zur Hälfte füllt und auf einer Temperatur von etwa 60° bis 70° C gehalten wird. Die obere Grenztemperatur darf kurzeitig bis zum 100° C betragen. Der Behälter 16 weist einen zentralen Durchgangskanal 16.0 auf. Dieser fluchtet mit der zentrischen Bohrung 24.1 innerhalb des Anschlußflansches 24 und ebenso mit der Achse des Zentralkanalstücks 15 innerhalb des Hohlzylinders 20. In diese zentrische Kanalflucht ist die schon erwähnte Fingerhuthülse 33 eingefügt, welche an ihrem oberen Ende federelastisch, wie beschrieben, arretiert ist und an ihrem unteren Ende aus der zentrischen Öffnung 16.0 des Behälters 16 um das Stück $a_1$ axial hervorschaut. In den Innenraum der Fingerhuthülse 33 ist der schon erwähnte Stabheizkörper 19 eingefügt und in der eingefügten Lage arretiert, wie noch erläutert wird. Da der Stabheizkörper praktisch am Innenumfang der Fingerhuthülse 33 anliegt, so strahlt die Fingerhuthülse 33 die Wärme direkt in das Zentralkanalstück 15 ab, wogegen der durch das Ringkanalstück 21 strömende erste Meßgasteilstrom indirekt über die zwischen beiden Kanalstücken 15, 21 befindliche Ringwand 20.2 beheizt wird. Der Stabheizkörper 19 kann im Bereich des Zentralkanalstückes 15 mit einer Heizwicklung versehen sein, die eine größere Wärmeleistung pro Längeneinheit abgibt als im Bereich der zentrischen Öffnung 16.0 des Behälters 16.

Der Behälter 16 weist wenigstens zwei zueinander konzentrisch angeordnete und durch eine ringförmige Zwischenwand 16.2 voneinander getrennte Teilkammern 16a und 16b zur Aufnahme des Filtermediums $B_0$ auf; seine äußere Zylinderwand ist mit 16.1, seine innere Zylinderwand mit 16.3 bezeichnet. Die beiden Teilkammer 16a, 16b sind in Reihe zueinander geschaltet, wobei die innere Teilkammer 16a an ihrem oberen Ende mit den zweiten internen Umleitkanälen 38 kommuniziert sowie an ihrem unteren Ende über Öffnungen 41 in der Zwischenwand 16 mit der äußeren Teilkamnmer 16b in Verbindung steht. Das Volumen der Teilkammer 16a ist etwa gleich dem Volumen der Teilkammer 16b, so daß bei der bevorzugten 50% Füllung und auftretenden Druckschwankungen keine Phosphorsäure aus dem Behälter 16 in die ankommenden oder abgehenden Meßgaskanäle gelangen kann. Die äußere Teilkammer 16b ist dann an ihrem oberen Ende über die dritten, anschlußflansch-internen Umleitkanäle 39 mit dem zweiten auslaßseitigen Anschluß kanal 40 verbunden, so daß der Teilstrom $r_{32}$ zunächst von oben nach unten strömt und dann von unten nach oben durch das Phosphorsäurebad $B_0$ perlt.

Der Behälter 16 ist mit den Oberkanten seiner ringförmigen Wände 16.1, 16.2, 16.3 gegen Dichtungsringe 42 entsprechend gestaffelten Durchmessers, welche in entsprechenden Ringnuten an der Unterseite des Anschlußflansches 24 sitzen, dichtend verspannbar.

Im folgenden wird diese Verspannung, welche unterhalb des Bodens des Behälters 16 angeordnet ist, näher erläutert. Die schon erwähnte Fingerhuthülse 33 trägt an ihrem unteren, offenen Ende an einem nicht näher bezeichneten Außengewinde eine Spannmutter 43, welche gegen einen am Boden 16.4 des Behälters 16 angreifenden hutförmigen Widerlager-Körper 44 axial verspannbar ist, wobei letzterer einen Innengewinde-Hals 44.1 zur Aufnahme der Spannmutter 43 und einen scheibenförmigen Basisteil 44.2 aufweist, der am Boden 16.4 des Behälters 16 anliegt, so daß beim Festziehen der Spannmutter 43 die Fingerhuthülse 33 unter Spannen der beiden Tellerfeder-Teilpakete 35.1, 35.2 ein geringes Stück nach unten gezogen und zugleich der hutförmige Widerlager-Körper 44 gegen den Boden 16.4 des Behälters 16 verspannt wird. Letzterer wird mithin mit den Oberkanten seiner ringförmigen Wände 16.11 bis 16.3 dichtend gegen die Dichtungsringe 42 gedrückt, wie bereits erläutert.

Die Spannmutter 43 ist an der Stelle 43.1 bezüglich der Fingerhuthülse 33 verdrehgesichert. Sie weist ein Innengewinde 43.2 auf, in welches der stabförmige Heizkörper 19 mit einem Außengewindehals 19.1 einschraubbar ist, zu welchem Zweck der Anschlußkopf 19.2 des Heizkörpers 19 einen Mehrkant 19.3 zum Ansetzen von Schraubenschlüsseln od.dgl. aufweist.

Die strömungsmäßige Verbindung zwischen dem Zentralkanalstück 15 und der ersten Teilkammer 16a des Behälters 16 erfolgt über die zweiten anschlußflanschinternen Umleitkanäle 38, wie bereits erwähnt. Diese bestehen aus der sich an das Zentralkanalstück 15 unmittelbar anschließenden zentralen Öffnung 24.1 welche einen Ringspalt zum Außenumfang der Fingerhuthülse 33 bildet, welcher geringfügig enger ist als die Ringspaltweite des Zentralkanalstücks 15. Hieran schließt sich eine erweiterte Ringkammer 38.1 an, deren Durchmesser so groß ist, daß die Axialbohrungen 38.2, welche in die erste Teilkammer 16a axial einmünden innerhalb des Bohrungsdurchmessers der Ringkammer 38.1 liegen.

Da die Mengen an $NH_3$ bzw. an der Komponente B kleiner sind als diejenigen der Komponente A kann der Strömungsquerschnitt des zweiten Meßkanals mk2 kleiner sein als derjenige des ersten Meßgaskanals mk1. Wenn in Fig. 2 auch nur zwei auslaßseitigen Anschlußkanäle 37 bzw. 40 dargestellt sind, so versteht es sich, daß über den Umfang des Anschlußflansches 24 verteilt noch weitere Anschlußkanäle 37 bzw 40 vorgesehen sein könnten Außerdem könnte zusätzlich zum oder anstelle des inneren Heizstab(es) eine Außenheizung vorgesehen sein. Bei reiner Außenheizung zum Beispiel durch eine Heizmuffe im Bereich des doppelwandigen Hohlzylinders 20, könnte der erste (innere) Meßgaskanal mk2 in seinem Durchmesser verkleinert werden.

Der Zwischenflansch 23 weist eine radiale Einstecköffnung 45 für wenigstens ein Temperatur-Meßelement 46 auf, dessen Meßkopf in das Ringkanalstück 21, d.h. in dessen Filtermasse $A_0$, ragt. Dargestellt ist eine Schraubhülse des Meßelementes 46, welche mittels Kontermutter 47 unter Zwischenlage eines Dichtungsringes 48 innerhalb der Einstecköffnung 45 dichtend festgelegt ist. Als Temperatur-Meßelemente kommen insbesondere Widerstands-Meßelememnte in Betracht.

Wie bereits angedeutet, weist die Gasentnahmesonde GES an der rohrförmigen Außenwand 20.1 ihres Hohlzylinders 20 im Bereich der ersten Hälfte von dessen axialer Länge einen Ringflansch 11 auf, mit welchem sie, mit ihrem Eintrittsende 17 in den Meßgasstutzen 7.3 eingesteckt (Fig. 1) an dem Gegenflansch 10 des Meßgasstutzens unter Einfügung eines Dichtungsringes 11.1 dichtend festgeschraubt werden kann.

Bei der Demontage der Gasentnahmesonde GES kann zweckmäßig so vorgegangen werden, daß zunächst der Stabheizkörper 19 losgeschraubt und herausgezogen wird. Nun können die Spannschrauben des Befestigungsflansches 11 gelöst und die Sonde aus dem Rauchgasentnahmestutzen entnommen werden. Diese Vorgänge werden in vertikaler Lage der Sonde vorgenommen, um ein Überlaufen der Phosphorsäure aus dem Behälter 16 zu vermeiden. Die Sonde kann in vertikaler Position mit ihrem Anschlußflansch 24 eingespannt, und es kann die Spannmutter 33.3 am oberen Ende gelöst werden. Man kann nun den noch gefüllten Behälter 16 nach unten zusammen mit der Fingerhuthülse 33 vorsichtig abziehen, entleeren und mit neuem Filtermaterial füllen. Weiterhin können an der Oberseite der Sonde die beiden ringscheibenförmigen Filterplatten 14 sowie 31 entfernt werden. Durch Auf-den-Kopf-Stellen des Hohlzylinders 20 kann die körnige Filtersubstanz $A_0$ ausgeschüttet werden. Zum Auswechseln der unteren Filterplatte 26 müssen die Flanschschrauben 30 gelöst werden. Nachdem die Flansche 23 und 24 wieder zusammengespannt sind, kann neue Filtersubstanz $A_0$ von oben wieder eingefüllt werden, wonach zweckmäßig entsprechend neue Filterplatten 31, 14 eingefügt werden.

Der Behälter 16, entleert von der verbrauchten flüssigen Filtersubstanz $B_0$ und wieder aufgefüllt mit neuer Filtersubstanz $B_0$ kann zusammen mit der Fingerhuthüse 33 von unten an den Anschlußflansch 24 wieder herangeführt und mit seiner Fingerhuthülse 33 durch dessen Bohrung hindurchgesteckt werden, wonach am oberen Ende die in Fig. 2 dargestellte Verschraubung wieder herbeigeführt wird, so daß die Fingerhuthülse 33 innerhalb des Zentralkanalstückes 15 zentrisch angeordnet ist. Als letztes kann dann der stabförmige Heizkörper 19 von unten in die Fingerhuthülse 33 eingesteckt und an der Spanmutter 43 verschraubt werden.

Im folgenden seien noch die wesentlichen chemischen Reaktionen erwähnt, die im Filter $A_0$ zur Rückhaltung des $SO_X$ und im Filter $B_0$ zur Rückhaltung des $NH_3$ führen.

Reaktionen im Filter $A_0$:

$$SO_2 + Na_2 CO_3 \rightarrow Na_2 SO_3 + CO_2$$
$$SO_3 + Na_2 CO_3 \rightarrow Na_2 SO_4 + CO_2$$
($Na_2 SO_3$ = Natriumsulfit; $Na_2 SO_4$ = Natriumsulfat).

Reaktionen im Filter $B_0$:

$$NH_3 + H_3 PO_4 \rightarrow (NH_4) H_2 PO_4$$
$$NH_3 + (NH_4) H_2 PO_4 \rightarrow (NH_4)_2 H PO_4$$
$(NH_4) H_2 PO_4$ = Ammoniumdihydrogenphosphat
$(NH_4)_2 H PO_4$ = Diammoniumhydrogenphosphat.

Abschließend sei noch ergänzend auf einige Vorteile hingewiesen, die mit der Erfindung erreichbar sind. Durch den innerhalb des Vorkammerabschnittes 7.1 angeordneten Zyklon 8 wird eine Grobstaubabscheidung, insbesondere der Ascheteilchen, erreicht und damit eine Schonung der nachgeschalteten Filterstrecken, bestehend aus dem Feinstaubfilter 9, dem Filter 14, der Filterplatte 31 (mechanische Filter) und der teilstromspezifischen chemischen Filter $A_0$ sowie $B_0$ innerhalb der Gasentnahmesonde GES. Dadurch sind wesentlich größere Standzeiten bzw. Wartungsintervalle für die genannten Filter und für die nachgeschalteten Gasmeßgeräte erreichbar.

Infolge der sehr hohen Strömungsgeschwindigkeit, welche der Zyklon 8 dem entnommenen Rauchgasstrom aufdrückt, ergeben sich sehr kurze Kontaktzeiten des Rauchgases. So wird z.B. eine unerwünschte Reaktion der im Rauchgas enthaltenen Gasanteile $NO_X$ und $NH_3$ am Staub, am Feinstaubfilter 9 (Keramikfilter) oder auch an der Rohrwandung weitgehend verhindert.

Mit Hilfe der beiden chemischen Filter $A_0$ und/oder $B_0$ kann die Genauigkeit bei der Nullpunkteinstellung und Kalibrierung der Meßgeräte wesentlich erhöht werden. Der Meßgas-Teilstrom $r_{32}$, welcher das chemische Filter $B_0$ verläßt, ist von der Komponente $NH_3$ befreit, und so kann dieser Meßgasteilstrom zur Nullpunkteinstellung von $NH_3$-Meßgeräten verwendet werden. Wird andererseits nach dem chemischen Filter $B_0$ über ein Zwischenstück (T-Stück) $NH_3$-Prüfgas in definierten Mengen zugegeben, so kann das $NH_3$-Meßgerät kalibriert werden.

Das entsprechende gilt für das andere chemische Filter $A_0$: Der Meßgas-Teilstrom $r_{31}$ ist bei in Betrieb befindlicher Gasentnahmesonde von $SO_X$ befreit, so daß dieser Teilstrom zur Nullpunkteinstellung von $SO_2$- oder $SO_3$-Meßgeräten verwendet werden kann. Andererseits kann eine definierte Menge $SO_X$ ($SO_2$ und/oder $SO_3$) über ein Zwischenstück nach dem chemischen Filter $A_0$ in die Meßgas leitung eingespeist werden, so daß nunmehr eine Kalibrierung der $SO_2$- oder $SO_3$-Meßgeräte vorgenommen werden kann.

Die Anwendung des Verfahrens nach der Erfindung in der Weise, daß die beiden chemischen Filter $A_0$ und $B_0$ in Reihe zueinander geschaltet sind, ist als Eich- oder Kalibrierschaltung besonders geeignet. Denn mit einer solchen Schaltung kann die Querempfindlichkeit des $NH_3$-Meßgerätes gegenüber $CO_2$ und $H_2O$ mit einkalibriert werden, gleichzeitig wird die Querempfindlichkeit gegenüber $SO_2$ eliminiert, weil kein $SO_2$ auf die Meßgeräte gelangen kann.

## Patentansprüche

1. Verfahren zur Reinhaltung der Meßleitungen an Emissions-Meßeinrichtungen von Rauchgas-Reinigungsanlagen, wobei der Meßgasstrom als gas- oder dampfförmige Verunreinigungen wenigstens eine Komponente (A) und eine Komponente (B) enthält, und Komponente (A) mit Komponente (B) unter Bildung flüssiger oder fester Niederschläge im Inneren der Meßleitungen reagiert, **dadurch gekennzeichnet,**
- daß der dem Rauchgas an einer einzigen Entnahmestelle entnommene Meßgasstrom ($r_3$) in wenigstens zwei parallele Teilströme ($r_{31}$, $r_{32}$) unterteilt wird und die beiden Teilströme auf eine Temperatur oberhalb des Kondensationspunktes der im Meßgas enthaltenen dampfförmigen Verunreinigungen (A, B) beheizt werden,
- daß der erste Teilstrom ($r_{31}$) durch ein erstes Filter ($A_0$) hindurchgeleitet wird, welches die Komponente (A) im Meßgasstrom selektiv herausfiltert,
- daß der zweite Teilstrom ($r_{32}$) durch ein zweites Filter ($B_0$) hindurchgeleitet wird, welches die Komponente (B) aus dem Meßgasstrom selektiv herausfiltert,
- und daß der gefilterte erste Teilstrom einer den Gehalt an der Komponente (B) und der gefilterte zweite Teilstrom einer den Gehalt an der Komponente (A) bestimmenden Emissions-Meßeinrichtung (AG1, AG2) zugeführt wird.

2. Verfahren nach Anspruch 1, zur Reinhaltung der Meßleitungen an Emissions-Meßeinrichtungen von Rauchgas-Entstickungsanlagen und/oder Rauchgas-Entschwefelungsanlagen, **dadurch gekennzeichnet,** daß der Meßgasstrom als gas- oder dampfförmige Verunreinigungen wenigstens $SO_X = SO_2 + SO_3$ als Komponente (A) und $NH_3$ als Komponente (B) enthält.

3. Verfahren nach Anspruch 1 oder 2, **dadurch gekennzeichnet**, daß die beiden Teilströme ($r_{31}$, $r_{32}$) auf ihrem Wege durch die Filter ($A_0$, $B_0$) beheizt werden.

4. Verfahren nach Anspruch 2 oder 3, **dadurch gekennzeichnet**, daß als Filtermaterial bzw. Absorptionsmittel für das Filter ($A_0$) des ersten Teilstroms ($r_{31}$) Soda $Na_2 CO_3$ in körniger, poröser oder Granulatform verwendet wird.

5. Verfahren nach Anspruch 4, **dadurch gekennzeichnet**, daß das Soda auf eine Betriebstemperatur von ca. 350° C erwärmt wird.

6. Verfahren nach einem der Ansprüche 2 bis 5, **dadurch gekennzeichnet**, daß als Filtermaterial bzw. Absorptionsmittel für das Filter ($B_0$) des zweiten Teilstroms ($r_{32}$) heiße Phosphorsäure, und zwar Orthophosphorsäure $H_3 PO_4$, verwendet wird.

7. Verfahren nach Anspruch 6, **dadurch gekennzeichnet**, daß die Phosphorsäure auf eine Betriebstemperatur von ca. 60 bis 70° C, maximal bis zu 100° C erwärmt wird.

8. Verfahren nach einem der Ansprüche 1 bis 7, **dadurch gekennzeichnet**, daß der dem Rauchgaskanal entnommene Rauchgasstrom vom größten Teil seines Staubes in wenigstens einem Zyklon befreit wird und eine Teilmenge ($r_3$) dieses vorgereinigten Rauchgasstromes als Meßgasstrom der Entnahmestelle zugeleitet wird.

9. Verfahren nach Anspruch 8, **dadurch gekennzeichnet**, daß der nicht als Meßgasstrom verwendete Anteil des vorgereinigten Rauchgasstromes über eine Bypaßleitung in den Rauchgaskanal (1) wieder zurückgefördert wird.

10. Verfahren nach einem der Ansprüche 1 bis 9, **dadurch gekennzeichnet**, daß der Meßgasstrom ($r_3$), bevor die Aufteilung in die wenigstens zwei Teilströme ($r_{31}$, $r_{32}$) vorgenommen wird, über eine Vorfilterstrecke geleitet wird.

11. Verfahren nach Anspruch 10, **dadurch gekennzeichnet**, daß der Meßgasstrom ($r_3$) über eine dem Zyklonausgang nachgeschaltete Entnahmelanze (6) und durch ein Feinstaubfilter (9) einer gemeinsamen Entnahmestelle (13) für beide Meßgas-Teilströme ($r_{31}$, $r_{32}$) und von hier über ein weiteres, gemeinsames Staubfilter (14) der gemeinsamen Einströmkammer (18), an deren Ausgang die Aufteilung auf die beiden Teilströme erfolgt, zugeleitet wird.

12. Anwendung des Verfahrens nach einem der Ansprüche 1 bis 11 auf die kondenswasserfreie Filterung von Meßgasen, die bei Rauchgas-Entstickungs- oder Rauchgas-Entschwefelungsanlagen anfallen, im Einweg-Filterverfahren, wobei $SO_X = SO_2 + SO_3$ aus dem Meßgas entfernt wird, indem es durch eine auf ca. 350° erwärmtes Soda als Filtermaterial enthaltende erste Filterstrecke geleitet wird, und/oder $NH_3$ aus dem Meßgas entfernt wird, indem es durch eine auf ca. 60° bis 70° C erwärmte Orthophosphorsäure als Filtermaterial enthaltende zweite Filterstrecke geleitet wird.

13. Gasentnahmesonde zur Durchführung des Verfahrens nach einem der Ansprüche 1 bis 11, zum Anschluß an Rauchgasleitungen als Vorschaltgerät für Emissions-Meßeinrichtungen, wobei der Meßgasstrom wenigstens eine erste gas- oder dampfförmige Komponente (A) und eine zweite gas- oder dampfförmige Komponente (B) als auszumessende Verunreinigungs-Komponenten enthält und Komponente (A) mit Komponente (B) unter Bildung flüssiger oder fester Niederschläge im Inneren der Meßleitungen reagiert, **dadurch gekennzeichnet**,

- daß die Gasentnahmesonde (GES) einen Hohlkörper mit wenigstens zwei internen, zueinander parallelgeschalteten ersten und zweiten Meßgaskanälen (mk1, mk2) sowie mit Gaseintritts- und Gasaustrittsöffnungen (mk11, mk21; mk12, mk22) an den beiden Enden der Meßgaskanäle aufweist,

- daß die Gasentnahmesonde mit einem Eintrittsende (17) ihres Hohlkörpers in den Gasstrom einer Rauchgasausströmzone eintauchend montierbar ist und innerhalb des Eintrittsendes eine Gaseintrittskammer (18) vorgesehen ist, welche auf ihrer Zuströmseite über wenigstens ein Staubfilter (14) mit der Rauchgasausströmzone und auf ihrer Abströmseite mit den Gaseintrittsöffnungen (mk11, mk21) beider Meßgaskanäle (mk1, mk2) kommuniziert;

- daß im ersten Meßgaskanal (mk1) ein erstes Filter ($A_0$) zum Herausfiltern der Komponente (A) und im zweiten Meßgaskanal (mk2) ein zweites Filter ($B_0$) zum Herausfiltern der Komponente (B) angeordnet ist und

- daß zumindest auf Teilstrecken die Strömungsräume und/oder Wandungen beider Meßgaskanäle (mk1, mk2) mit einer Heizeinrichtung (19) in wärmeübertragendem Kontakt stehen, welche die durch die Maßgaskanäle strömenden Teilströme ($r_{31}$, $r_{32}$) auf einer Temperatur oberhalb der höchsten Kondensationstemperatur der zu filternden Komponenten (A bzw. B) hält.

14. Gasentnahmesonde nach Anspruch 13, **dadurch gekennzeichnet**, daß der Hohlkörper einen doppelwandigen Hohlzylinder (20) aufweist, mit einem Ringkanalstück (21) und einem Zentralkanalstück (15) als durch eine Ringwand (20.2) voneinander getrennte und zueinander parallel und konzentrisch verlaufende Meßgaskanalstrecke des ersten bzw. des zweiten Meßgaskanals (mk1, mk2)

- daß der Hohlkörper mit seinem Ringkanalstück (21) eine Filterstrecke des ersten Meßgaskanals (mk1) bildet und in einem dichtend mit ihm verbundenen Anschlußflansch (24) endet sowie mit einem abgehenden ersten auslaßseitigen Anschlußkanal (37) des Anschlußflansches (24) über erste interne Umleitkanäle (24.2) kommuniziert,

- daß auch das Zentralkanalstück (15) in einem Anschlußflansch (24) endet und über zweite interne Umleitkanäle (38; 24.1, 38.1, 38.2) des Anschlußflansches (24) in eine Filterstrecke ($B_0$) des zweiten Meß-

gaskanals (mk2) übergeht, welche auf der dem doppelwandigen Hohlzylinder (20) abgewandten Seite des Anschlußflansches (24) innerhalb eines hohlzylindrischen, dichtend mit dem Anschlußflansch verbundenen Behälters (16) angeordnet ist,
- daß der Behälter (16) in seinem geodätisch am höchstem liegenden Bereich über dritte anschlußflanschinterne Umleitkanäle (39) mit einem zweiten abgehenden auslaßseitigen Anschlußkanal (40) des Anschlußflansches (24) kommuniziert.

15. Gasentnahmesonde nach Anspruch 14, **dadurch gekennzeichnet**, daß ein Durchgangskanal (16.0) des Behälters (16) mit einer zentrischen Öffnung in dem Anschlußflansch (24) bzw. den Anschlußflanschen und mit dem Zentralkanalstück (15) des Hohlzylinders (20) fluchtet und in den so gebildeten hohlkörperaxialen Aufnahmekanal wenigstens ein stabförmiges Heizelement (19) eingefügt ist, dessen Mantelfläche den durch das Zentralkanalstück (15) strömenden zweiten Meßgasteilstrom (r32) direkt und den durch das Ringkanalstück (21) strömenden ersten Meßgasteilstrom (r31) indirekt über die zwischen beiden Kanalstücken befindliche Ringwand (20.2) beheizt.

16. Gasentnahmesonde nach Anspruch 14, **dadurch gekennzeichnet**, daß ein Anschlußflansch (24) und ein axial dazu beabstandeter Zwischenflansch (23) unter Einfügung eines hohlzylindrischen Schauglaskörpers (27) miteinander dichtend verspannt sind, und daß der Anschlußflansch (24) die flanschinternen Umleitkanäle (24.2, 38, 39) und die Anschlußkanäle (37, 40) des ersten und des zweiten Meßgaskanals (mk1, mk2) enthält.

17. Gasentnahmesonde nach Anspruch 16, **dadurch gekennzeichnet**, daß der Zwischenflansch (23) eine Einstecköffnung (45) für wenigstens ein Temperatur-Meßelement (46) aufweist, dessen Meßkopf in das Ringkanalstück (21) ragt.

18. Gasentnahmesonde nach Anspruch 16, **dadurch gekennzeichnet**, daß zwischen der dem Anschlußflansch (24) zugewandten Stirnseite des Schauglaskörpers (27) und dem genannten Anschlußflansch wenigstens eine scheibenförmige Filterplatte (26) in eine entsprechende Ringaussparung unter Abdeckung der ersten internen Umleitkanäle (24.2) eingesetzt ist, so daß das Ringkanalstück (21) über diese Filterplatte (26) mit den ersten internen Umleitkanälen (24.2) kommuniziert.

19. Gasentnahmesonde nach Anspruch 14, **dadurch gekennzeichnet**, daß der vorzugsweise aus Glas bestehende Behälter (16) für die Filterstrecke (B0) des zweiten Meßgaskanals (mk2) wenigstens zwei zuein ander konzentrisch angeordnete und durch eine ringförmige Zwischenwand (16.2) voneinander getrennte Teilkammern (16a, 16b) zur Aufnahme des Filtermediums aufweist.

20. Gasentnahmesonde nach Anspruch 19, **dadurch gekennzeichnet**, daß die beiden Teilkammern (16a, 16b) in Reihe zueinander geschaltet sind und eine innere Teilkammer (16a) an ihrem oberen Ende mit den zweiten internen Umleitkanälen (38) kommuniziert sowie an ihrem unteren Ende über Öffnungen (41) in der Zwischenwand (16.2) mit der äußeren Teilkammer (16b) in Verbindung steht, wobei die äußere Teilkammer (16b) an ihrem oberen Ende über die dritten anschlußflanschinternen Umleitkanäle (39) mit dem zweiten auslaßseitigen Anschlußkanal (40)) kommuniziert.

21. Gasentnahmesonde nach dem Anspruch 15, **dadurch gekennzeichnet**, daß eine den Durchgangskanal des Behälters (16) und den Zentralkanal(15, 16.0) zentrisch durchdringende Fingerhuthülse (33) zum Einstecken des stabförmigen Heizkörpers (19) vorgesehen ist, welche am Eintrittsende (17) des Hohlkörpers mit ihrem einen Boden (33.1) aufweisenden Ende verankert ist und am anderen offenen Ende an einem Außengewinde eine Spannmutter (43) trägt, welche gegen einen am Boden (16.4) des Behälters (16) angreifenden hutförmigen Widerlager-Körper (44) axial verspannbar ist, wobei letzterer einen Innengewinde-Hals (44.1) zur Aufnahme der Spannmutter (43) und einen scheibenförmigen Basisteil (44.2) aufweist, mittels welchem der Behälter (16) mit den Oberkanten seiner ringförmigen Wände (16.1, 16.2, 16.3) gegen Dichtungsringe (42) dichtend verspannbar ist, die in entsprechende Ringnuten der Unterseite des Anschlußflansches (24) eingelegt sind.

22. Gasentnahmesonde nach einem der Ansprüche 13 bis 21, mit einer körnigen Schüttung als Filterstrecke für den ersten Meßgaskanal (mk1), **dadurch gekennzeichnet**, daß die Gaseintrittsöffnung (mk11) des ersten Meßgaskanals (mk1) von einer de- und remontablen Filterplatte (31) abgedeckt ist.

## Claims

1. Method for keeping clean the measuring tubes of emission-measuring apparatuses of flue gas purifying installations, wherein the stream of gas for analysis contains as gaseous or vapour impurities at least one component (A) and one component (B), and component (A) reacts with component (B) to form liquid or solid deposits on the inside of the measuring tubes, characterised in that
- the stream of gas for analysis (r3) taken from the flue gas at a single sampling point is sub-divided into at least two parallel branch streams (r31, r32) and the two branch streams are heated to a temperature above the condensation point of the vapour impurities (A, B) contained in the gas for analysis,
- the first branch stream (r31) is passed through a first filter (A0) which selectively filters out component (A) in the stream of gas for analysis,
- the second branch stream (r32) is passed through a second filter (B0) which selectively filters out component (B) from the stream of gas for analysis,

– and the filtered first branch stream is conducted to an emission-measuring apparatus (AG1, AG2) which determines the amount of component (B), and the filtered second branch stream to one which determine the amount of component (A).

2. Process according to claim 1, for keeping clean the measuring tubes of emission-measuring apparatuses of flue-gas nitrogen removal installations and/or of fluegas sulphur removal installations, characterised in that as gaseous or vapour impurities the stream of gas for analysis contains at least $SO_x = SO_2 + SO_3$ as component (A) and $NH_3$ as component (B).

3. Process according to claim 1 or 2, characterised in that the two branch streams ($r_{31}$, $r_{32}$) are heated en route through the filters ($A_0$, $B_0$).

4. Process according to claim 2 or 3, characterised by using soda $Na_2CO_3$ in gritty, porous or granulate form as the filter material or absorption material for the filter ($A_0$) of the first branch stream ($r_{31}$).

5. Process according to claim 4, characterised by heating the soda to an operating temperature of around 350°C.

6. Process according to one of claims 2 to 5, characterised by using hot phosphoric acid, specifically orthophosphoric acid $H_3PO_4$, as the filter material or absorption material for the filter ($B_0$) of the second branch stream ($r_{32}$).

7. Process according to claim 6, characterised by heating the phosphoric acid to an operating temperature of around 60 to 70°C, but not more than 100°C.

8. Process according to one of claims 1 to 7, characterised in that at least one cyclone removes most of the dust from the flue gas stream taken from the flue gas condut and a portion ($r_3$) of this precleaned flue gas stream is conducted to the sampling point as the stream of gas for analysis.

9. Process according to claim 8, characterised in that the portion of the pre-cleaned flue gas stream not used as the stream of gas for analysis is carried back into the flue gas conduit (1) along a bypass

10. Process according to one of claims 1 to 9, characterised in that the stream of gas for analysis ($r_3$), before being divided into the not less than two branch streams ($r_{31}$, $r_{32}$), is fed through a prefiltering section.

11. Process according to claim 10, characterised in that the stream of gas for analysis ($r_3$) is fed via a sampling lance (6), connected downstream of the cyclone exit, and through a fine dust filter (9) to a common sampling point (13) for the two branch streams ($r_{31}$, $r_{32}$) of the gas for analysis, and from there via a further, common dust filter (14) to the common admission chamber (18), at the exit of which the division into the two branch streams takes place.

12. Use of the process according to one of claims 1 to 11 for filtering, without condensation water, gases for analysis which are produced in flue-gas nitrogen or flue-gas sulphur removal installations, in a one-way filtering process, wherein $SO_x = SO_2 + SO_3$ is removed from the gas for analysis by feeding it through a first filter section containing soda heated to around 350°C as the filter material, and/or $NH_3$, is removed from the gas for analysis by feeding it through a second filter section containing orthophosphoric acid heated to around 60 to 70°C as the filter material.

13. Gas sampling probe for carrying out the process according to one of claims 1 to 11, for connection to flue-gas pipes as a control device for emission-measuring apparatuses, wherein the stream of gas for analysis contains at least one first gaseous or vapour component (A) and one second gaseous or vapour component (B) as the impurity components to be measured and component (A) reacts with component (B) to form liquid or solid deposits on the inside of the measuring tubes, characterised in that

– the gas sampling probe (GES) has a hollow body with at least two internal first and second analysis-gas conduits (mk1, mk2) arranged parallel to one another and with gas admission and gas discharge ports (mk11, mk21; mk12, mk22) at both ends of the analysis-gas conduits,

– the gas sampling probe is adapted to be assembled with one admission end (17) of its hollow body immersed in the gas stream of a flue-gas outflow zone, and a gas admission chamber (18) is provided inside the admission end, said chamber communicating on its inflow side via at least one dust filter (14) with the flue-gas outflow zone and on its outflow side with the gas admission ports (mk11, mK21) of the two analysis-gas conduits (mk1, mk2);

– in the first analysis-gas conduit (mk1) there is arranged a first filter ($A_0$) for filtering out component (A) and in the second analysis-gas conduit (mk2) there is arranged a second filter ($B_0$) for filtering out component (B), and

– at least along sub-sections the flow compartments and/or wall; of the two analysis-gas conduits (mk1, mk2) are in heat-exchanging contact with a heating device (19) which keeps the branch streams ($r_{31}$, $r_{32}$) which flow through the analysis-gas conduits at a temperature above the highest condensation temperature of the components (A or B) being filtered.

14. Gas sampling probe according to claim 13, characterised in that the hollow body has a double-walled hollow cylinder (30), with an annular conduit element (21) and a central conduit element (15) as the analysis-gas conduit section of respectively the first or second analysis-gas conduit (mk1, mk2), which are separated from one another by an annular wall (20.2) and run parallel and concentric to one another,

– the hollow body forms with its annular conduit element (21) a filter section of the first analysis-gas conduit (mk1) and terminates in a connecting flange (24) joined sealingly thereto, and communicates with an outgoing first outlet-side connecting conduit (37) of the connecting flange (24) by means of first internal bypass conduits (24.2),
- the central conduit section (15) likewise terminates in a connecting flange (24) and merges via second internal bypass conduits (38; 24.1, 38.1, 38.2) of the connecting flange (24) into a filter section (B₀) f the second analysis-gas conduit (mk2), said filter section being disposed on the side of the connecting flange (24) that is remote from the double-walled hollow cylinder (20), inside a hollow-cylindrical container (16) joined sealingly to the connecting flange,
- the container (16) communicates in its geodetically most elevated area with a second outgoing outlet-side connecting conduit (40) of the connecting flange (24) via third bypass conduits (39) internal to the connecting flange.

15. Gas sampling probe according to claim 14, characterised in that a passage (16.0) in the container (16) is flush with a central opening in the connecting flange (24) or connecting flanges and with the central conduit element (15) of the hollow cylinder (20) and at least one rod-shaped heating element (19) is inserted into the thus constituted receiving conduit axially aligned in the hollow body plane, the generated surface of said heating element directly heating the second branch stream of analysis gas (r₃₂) which flows through the central conduit element (15) and indirectly heating the first branch stream of analysis gas (r₃₁) which flows through the annular conduit element (21) by the agency of the annular wall (20.2) situated between the two conduit elements.

16. Gas sampling probe according to claim 14, characterised in that a connecting flange (24) and an intermediate flange (23) spaced axially apart therefrom are sealingly tensioned with one another, a hollow-cylindrical glass inspection body (27) being interposed between them, and that the connecting flange (24) contains the bypass conduits (24.2, 38, 39) internal to the flange and the connecting conduits (37, 40) of the first and second analysis-gas conduit (mk1, mk2).

17. Gas sampling probe according to claim 16, characterised in that the intermediate flange (23) has an insert opening (45) for at least one temperature measuring element (46), the measuring head of which projects into the annular conduit element (21).

18. Gas sampling probe according to claim 16, characterised in that between the end face of the glass in body (27) nearest the connecting flange (24) and the said connecting flange, at least one discoid filter plate (26) is inserted into a corresponding annular recess so as to obturate the first internal bypass conduits (24.2), with the result that the annular conduit element (21) communicates with the first internal bypass conduits (24.2) via this filter plate (26).

19. Gas sampling probe according to claim 14, characterised in that the preferably glass container (16) for the filter section (B₀) of the second analysis-gas conduit (mk2) has at least two sub-chambers (16a, 16b) arranged concentrically to one another and separated from one another by an annular partition (16.2), for receiving the filter medium.

20. Gas sampling probe according to claim 19, characterised in that the two sub-chambers (16a, 16b) are connected in series and an inner sub-chamber (16a) communicates at its top end with the second internal bypass conduits (38) and is connected at its bottom end, via openings (41) in the partition (16.2), to the outer sub-chamber (16b), the outer sub-chamber (16b) communicating at its top end with the second outlet-side connecting conduit (40) via the third bypass conduits (39) internal to the connecting flange.

21. Gas sampling probe according to claim 15, characterised in that there is provided a thimble-type sleeve (33) passing centrally through the passage in the container (16) and through the central conduit (15, 16.0) for inserting the rod-shaped heating element (19), which is anchored at the admission end (17) of the hollow body by its end having a plate (33.1) and at the other open end carries a tensioning nut (43) on a male thread, said nut being adapted to be axially tensioned against a cap-shaped abutment body (44) engaging on the base (16.4) of the container (16), said abutment body having a female thread neck (44.1) for receiving the tensioning nut (43) and a discoid base portion (44.2), by means of which the container (16) can be sealingly tensioned by the top edges of its annular walls (16.1, 16.2, 16.3) against sealing rings (42) positioned in corresponding annular grooves on the bottom face of the connecting flange (24).

22. Gas sampling probe according to one of claims 13 to 21, with a gritty bulk material as the filter section for the first analysis-gas conduit (mk1), characterised in that the gas admission port (mk11) of the first analysis-gas conduit (mk1) is obturated by a demountable and remountable filter plate (31).

**Revendications**

1. Procédé pour tenir propres les conduits de mesure de dispositifs de mesure de l'émission d'installations d'épuration de gaz de fumée, le courant de gaz à mesurer contenant comme impuretés, sous forme de gaz ou de vapeur, au moins un constituant (A) et un constituant (B), et le constituant (A) réagissant avec le constituant (B) en formant des dépôts liquides ou solides à l'intérieur des conduits de mesure, caractérisé en ce que,
- le courant de gaz à mesurer (r₃), prélevé du gaz de fumée en un emplacement de prélèvement unique, est subdivisé en au moins deux courants partiels (r₃₁, r₃₂) parallèles et les deux courants partiels sont portés à une température supérieure au point de condensation des impuretés (A, B) sous forme de vapeur contenues dans le gaz à mesurer,

– le premier courant partiel ($r_{31}$) est envoyé dans un premier filtre ($A_0$) qui filtre sélectivement le constituant (A) du courant de gaz à mesurer,

– le second courant partiel ($r_{32}$) est envoyé dans un second filtre ($B_0$) qui filtre sélectivement le constituant (B) du courant de gaz à mesurer,

– et le premier courant partiel filtré est envoyé à un dispositif de mesure de l'émission (AG1) déterminant la teneur en le constituant (B) et le second courant partiel filtré est envoyé à un dispositif de mesure de l'émission (AG2) déterminant la teneur en le constituant (A).

2. Procédé suivant la revendication 1, pour tenir propres les conduits de mesure de dispositifs de mesure de l'émission d'installations de dénitruration de gaz de fumée et/ou d'installations de désulfuration de gaz de fumée, caractérisé en ce que le courant de gaz à mesurer contient, comme impuretés sous forme de gaz ou sous forme de vapeur, au moins SOX, x = $SO_2$ + $SO_3$, comme constituant (A) et $NH_3$ comme constituant (B).

3. Procédé suivant la revendication 1 ou 2, caractérisé en ce que les deux courants partiels ($r_{31}$, $r_{32}$) sont chauffés sur leur trajet dans les filtres ($A_0$, $B_0$).

4. Procédé suivant la revendication 2 ou 3, caractérisé en ce qu'il consiste à utiliser, comme matière filtrante ou comme agent d'absorption pour le filtre ($A_0$) du premier courant partiel ($r_{31}$), du carbonate de sodium $Na_2 CO_3$, en grains, poreux ou sous forme de granulée.

5. Procédé suivant la revendication 4, caractérisé en ce qu'il consiste à chauffer le carbonate de sodium à une température de fonctionnement de 350°C environ.

6. Procédé suivant l'une des revendications 2 à 5, caractérisé en ce qu'il consiste à utiliser, comme matière filtrante ou comme agent d'absorption pour le filtre ($B_0$) du second courant partiel ($r_{32}$), de l'acide phosphorique chaud, et de fait de l'acide orthophosphorique $H_3 PO_4$.

7. Procédé suivant la revendication 6, caractérisé en ce qu'il consiste à chauffer l'acide phosphorique à une température de fonctionnement de 60 à 70°C environ et allant au maximum jusqu'à 100°C.

8. Procédé suivant l'une des revendications 1 à 7, caractérisé en ce qu'il consiste à débarrasser le courant de gaz de fumée, prélevé du canal de gaz de fumée, de la plus grande partie de sa poussière dans au moins un cyclone et à envoyer une quantité partielle ($r_3$) de ce courant de gaz de fumée pré-épurée, à titre de courant de gaz à mesurer, à l'emplacement de prélèvement.

9. Procédé suivant la revendication 8, caractérisé en ce qu'il consiste à retourner la partie du courant de gaz de fumée pré-épurée, qui n'a pas été utilisée comme courant de gaz à mesurer, par un conduit de dérivation, au canal pour le gaz de fumée (1).

10. Procédé suivant l'une des revendication 1 à 9, caractérisé en ce qu'il consiste à envoyer le courant de gaz à mesurer ($r_3$), avant d'effectuer la répartition en au moins deux courants partiels ($r_{31}$, $r_{32}$), sur une zone de préfiltration.

11. Procédé suivant la revendication 10, caractérisé en ce qu'il consiste à envoyer le courant de gaz à mesurer ($r_3$), par l'intermédiaire d'une lance de prélèvement (6) montée en aval de la sortie du cyclone et par l'intermédiaire d'un filtre de poussière fine (9), à un emplacement commun de prélèvement (13) des deux courants partiels de gaz à mesurer ($r_{31}$, $r_{32}$) et, de là, par un autre filtre à poussière (14) commun, à la chambre d'entrée (18) commune, à la sortie de laquelle s'effectue la subdivision en les deux courants partiels.

12. Utilisation du procédé suivant l'une des revendications 1 à 11, pour la filtration, sans eau de condensation, de gaz à mesurer qui se forment dans des installations de dénitruration du gaz de fumée ou de désulfuration du gaz de fumée, par un procédé de filtration à trajet unique, $SO_x$ = $SO_2$ + $SO_3$ étant éliminé du gaz à mesurer, en l'envoyant dans une première zone de filtration chauffée à 350°C environ et contenant du carbonate de sodium comme matière filtrante et/ou le $NH_3$ étant éliminé du gaz à mesurer en l'envoyant dans une seconde zone filtrante chauffée entre 60° et 70°C environ et contenant de l'acide orthophosphorique comme matière filtrante.

13. Sonde de prélèvement de gaz pour la mise en œuvre du procédé suivant l'une des revendications 1 à 11, destinée à être raccordée au conduit de gaz de fumée en tant qu'appareil monté en amont de dispositifs de mesure de l'émission, le courant de gaz à mesurer contenant au moins un premier constituant (A) sous forme de gaz ou de vapeur et un second constituant (B) sous forme de gaz ou de vapeur, comme constituants d'impuretés à mesurer, et le constituant (A) réagissant avec le constituant (B) en formant des dépôts liquides ou solides à l'intérieur des conduits de mesure, caractérisée en ce que,

– la sonde de prélèvement du gaz (GES) comporte un corps creux ayant au moins deux premier et second canaux pour le gaz à mesurer (mk1, mk2), internes et montés en parallèle l'un avec l'autre, ainsi que des orifices d'entrée et de sortie pour le gaz (mk11, mk21 ; mk12, mk22) aux deux extrémités des canaux pour le gaz à mesurer,

– la sonde de prélèvement du gaz peut être montée de manière à pénétrer, par une extrémité d'entrée (17) de son corps creux, dans le courant gazeux d'une zone de sortie du gaz de fumée, et dans l'extrémité d'entrée, est prévue une chambre d'entrée pour le gaz (18) qui communique, par son côté d'arrivée du courant, par au moins un filtre à poussière (14), avec la zone de sortie du gaz de fumée et, par son côté de sortie du courant, avec les orifices d'entrée pour le gaz (mk11, mk21) des deux canaux pour le gaz à mesurer (mk1, mk2);

– dans le premier canal pour le gaz à mesurer (mk1) est monté un premier filtre ($A_0$) pour enlever le cons-

tituant (A) par filtration et, dans le second canal pour le gaz à mesurer (mk2), un second filtre (B₀) pour enlever le constituant (B) par filtration, et
– au moins sur des zones partielles, les chambres d'écoulement et/ou les parois des deux canaux pour le gaz à mesurer (mk1, mk2) sont en contact d'échange thermique avec un dispositif de chauffage (19) qui maintient des courants partiels (r₃₁, r₃₂) passant par les canaux pour le gaz à mesurer, à une température supérieure à la température de condensation la plus élevée des constituants (A et B) à filtrer.

14. Sonde de prélèvement de gaz suivant la revendication 13, caractérisée en ce que le corps creux comporte un cylindre creux (20) à double paroi, ayant une pièce formant canal annulaire (21) et une pièce formant canal central (15), servant de zone du premier et du second canal pour le gaz à mesurer (mk1, mk2), s'étendant parallèlement l'une à l'autre et concentriquement et séparées l'une de l'autre par une paroi annulaire (20.2),
– en ce que le corps creux, avec sa pièce formant le canal annulaire (21) forme une zone de filtration du premier canal pour le gaz à mesurer (mk1) et se termine dans une bride de raccordement (24) qui est reliée avec celui-ci, de manière étanche, et communique, par de premiers canaux internes de déviation (24.2), avec un premier canal de raccordement (37), sortant et disposé du côté sortie, de la bride de raccordement (24),
– en ce que la pièce formant le canal central (15) se termine également dans une bride de raccordement (24) et se transforme, par l'intermédiaire de seconds canaux internes de déviation (38; 24.1, 38.1, 38.2) de la bride de raccordement (24), en une zone de filtration (B₀) du second canal pour le gaz à mesurer (mk2), qui est disposée du côté de la bride de raccordement (24) éloigné du cylindre creux (20) à double paroi, dans une cuve (16) en forme de cylindre creux reliée de manière étanche à la bride de raccordement,
– en ce que la cuve (16) communique, dans sa partie géodésique la plus élevée, par l'intermédiaire de troisièmes canaux de déviation (39) internes à la bride de raccordement, avec un second canal de raccordement (40) d'évacuation, se trouvant du côté sortie de la bride de raccordement (24).

15. Sonde de prélèvement de gaz suivant la revendication 14, caractérisée en ce qu'un canal de passage (16.0) de la cuve (16) est aligné avec une ouverture centrale de la bride de raccordement (24) ou des brides de raccordement, et avec la pièce formant le canal central (15) du cylindre creux (20) et dans le canal de réception axial creux ainsi formé, est insérée au moins une barre de chauffage (19) dont la surface latérale chauffe directement le courant partiel de gaz à mesurer (r₃₂) passant dans la pièce formant le canal central (15) et, indirectement par l'intermédiaire de la paroi annulaire (20.2) se trouvant entre les deux pièces formant canaux, le premier courant partiel de gaz à mesurer (r₃₁) passant dans la pièce formant le canal annulaire (21).

16. Sonde de prélèvement de gaz suivant la revendication 14, caractérisée en ce qu'une bride de raccordement (24) et une bride intermédiaire (23) à distance axiale de celle-ci, sont serrées l'une contre l'autre de manière étanche, avec interposition d'un hublot (27) en forme de cylindre creux, et en ce que la bride de raccordement (24) comporte les canaux de déviation (24.2, 38, 39) internes à la bride et les canaux de raccordement (37, 40) du premier et du second canal pour le gaz à mesurer (mk1, mk2).

17. Sonde de prélèvement de gaz suivant la revendication 16, caractérisée en ce que la bride intermédiaire (23) comporte une ouverture d'enfichage (45) destinée à au moins un élément de mesure de la température (46), dont la tête de mesure fait saillie dans la pièce formant le canal annulaire (21).

18. Sonde de prélèvement de gaz suivant la revendication 16, caractérisée en ce que, entre le côté frontal du hublot (27) qui est tourné vers la bride de raccordement (24) et ladite bride de raccordement, au moins une plaque de filtre (26) en forme de disque est insérée dans un évidement annulaire correspondant, en recouvrant les premiers canaux internes de déviation (24.2), de sorte que la pièce formant le canal annulaire (21) communique par cette plaque de filtre (26) avec les premiers canaux internes de déviation (24.2).

19. Sonde de prélèvement de gaz suivant la revendication 14, caractérisée en ce que la cuve (16), de préférence en verre, comporte pour la zone de filtration (B0) du second canal pour le gaz à mesurer (mk2), au moins deux chambres partielles (16a, 16b) de réception de l'agent filtrant, qui sont disposées concentriquement l'une à l'autre et qui sont séparées l'une de l'autre par une paroi intermédiaire (16.2) annulaire.

20. Sonde de prélèvement de gaz suivant la revendication 19, caractérisée en ce que les deux chambres partielles (16a, 16b) sont montées en série et une chambre partielle intérieure (16a) communique, par son extrémité supérieure, avec les seconds canaux internes de déviation (38), ainsi que, par son extrémité inférieure et par des orifices (41) de la paroi intermédiaire (16.2), avec la chambre partielle extérieure (16b), la chambre partielle extérieure (16b) communiquant, par son extrémité supérieure et par l'intermédiaire des troisièmes canaux de déviation (39) internes à la bride de raccordement, avec le second canal de raccordement (40) du côté sortie.

21. Sonde de prélèvement de gaz suivant la revendication 15, caractérisée en ce qu'il est prévu une douille en doigt de gant (33) passant dans la partie centrale du canal de traversée de la cuve (16) et du canal central (15, 16.0) et destinée à enfiler la barre de chauffage (19) qui est ancrée à l'extrémité d'entrée (17) du corps creux par son extrémité tournée vers un fond (33.1) comportant à l'autre extrémité ouverte, sur un filetage, un écrou de blocage (43) qui peut être bloqué axialement contre une pièce de contre-appui (44) en forme de capuchon appliquée au fond (16.4) de la cuve, cette dernière pièce comportant un collet taraudé (44.1) de réception de l'écrou de blocage (43) et une pièce de base (44.2) en forme de disque, au moyen de laquelle la cuve (16) peut être serrée de manière étanche par les bords supérieurs de ses parois annulaires (16.1, 16.2, 16.3) sur des bagues d'étanchéité (42) qui sont logées dans des gorges annulaires correspondantes ménagées dans le côté inférieur de la bride de raccordement (24).

22. Sonde de prélèvement de gaz suivant l'une des revendications 13 à 21, comprenant un amas de grains comme zone de filtration pour le premier canal du gaz à mesurer (mk1), caractérisée en ce que l'orifice d'entrée du gaz (mk1) du premier canal pour le gaz à mesurer (mk1) est recouvert d'une plaque de filtre (31) démontable et remontable.

FIG 1

EP 0 239 744 B1

FIG 2

| FIG 2A |
| FIG 2B |

FIG 2A

FIG 2B